Europäisches Patentamt

European Patent Office

Office européen des brevets

(19)

(11) Publication number: **0 322 477**
**A1**

# EUROPEAN PATENT APPLICATION

(21) Application number: **87119335.5**

(22) Date of filing: **29.12.87**

(51) Int. Cl.4: **G01N 21/51**

(43) Date of publication of application:
**05.07.89 Bulletin 89/27**

(84) Designated Contracting States:
**DE FR GB**

(71) Applicant: **Inoue, Hiroaki**
**1-10-1-1305, Meinohama Nishi-ku**
**Fukuoka-shi Fukuoka-ken(JP)**

(72) Inventor: **Inoue, Hiroaki**
**1-10-1-1305, Meinohama Nishi-ku**
**Fukuoka-shi Fukuoka-ken(JP)**

(74) Representative: **Schön, Alfred, Dr. et al**
**Patentanwälte Müller-Boré, Deufel, Schön,**
**Hertel Lewald, Otto Isartorplatz 6 Postfach 26**
**02 47**
**D-8000 München 26(DE)**

(54) Method for evaluating physiological activity of substances and apparatus for the same.

(57) The method comprises placing test solutions and a buffer solution separately in a cell made of a material that is pervious to laser rays; transmitting a laser beam through each of the solutions in the cell while it is exposed to thermal radiation from an object held at a temperature higher than that of the test solution; measuring expansion of laser beam for measuring degree of fluctuation in liquid structure, by the use of horizontal position of photoreceiver at the rise part of scattering profiles; plotting the relative position of a photoreceiver against the concentration of substance being tested; and evaluating the physiological activity of the substance from the pattern of graph prepared above.

An apparatus for evaluating physiological activity is also claimed.

## FIG. 1

## Method for Evaluating Physiological Activity of Substances and Apparatus for the Same

### BACKGROUND OF THE INVENTION

A great variety of physiologically active substances are now used for various purposes, including those to be directly administered to, and absorbed by, humans, animals and plants, such as medicines and agricultural chemicals. In order to evaluate the physiological activity of a agricultural chemicals, for example, it is common practice to actually applied to plants for absorption, and those skilled in the art observe any physiological changes and judge the effectiveness and toxicity.

On the other hand, with medicine for humans, it is first administered to test animals ranging from rats, mice and guinea pigs to cats, dogs and monkeys and their physiological changes are observed, thereby judging the effectiveness and toxicity.

I formerly developed a technique to evaluate the physiological activity of a substance while putting it in a state similar to that in living bodies, which comprises placing a liquid sample containing water in a cell made of a material pervious to laser rays; giving a minute stream of heat to said liquid sample and supplying conductive and radiation heat from a surrounding object; allowing a laser beam to pass through the sample in the cell; recording the change in intensity of the resultant scattered light with time on a fixed phototransistor; and evaluating the physiological activity of said substance from the pattern of this change [Japanese Patent Kokai No.130856 (1986)].

### SUMMARY OF THE INVENTION

This invention relates to a method for evaluating the physiological activity of a substance by putting said substance in a state similar to that as in a living body and by measuring the fluctuations in the solution structure, and to an apparatus to be directly used for this purpose.

The method and apparatus of this invention allow correct evaluation of physiological activity and toxicity of substances by simple operation.

Developmental work of a new substance, if judged as toxic by the method and apparatus of this invention, can be stopped or properly controlled without delay, thereby significantly saving costs and labor for development.

The invention will be better understood from the following description.

### BRIEF DESCRIPTION OF THE DRAWINGS

Figure 1 is a longitudinal sectional view of the apparatus of this invention.

Figure 2 is a transverse sectional view of the apparatus of this invention.

Figure 3(a) is a light scattering profile for $\beta$-indoleacetic acid on an X-Y recorder prepared in Example 1(a) by the method of this invention.

Figure 3(b) is a pattern of $\beta$-indoleacetic acid for evaluating its physiological activity.

Figure 4 shows Thimann's curves illustrating the physiological activity of $\beta$-indoleacetic acid (growth acceleration and retardation).

Figure 5 is a pattern of indolecarboxylic acid for evaluating its physiological activity prepared in Example 1(a) by the method of this invention.

Figure 6 is a pattern of 2,4,5-T for evaluating its physiological activity prepared in Example 2 by the method of this invention.

Figure 7 is a pattern of 2,4,6-T for evaluating its physiological activity prepared in Example 2 by the method of this invention.

Figure 8 is a pattern of $\alpha$-naphthaleneacetic acid for evaluating its physiological activity prepared in Example 3 by the method of this invention.

Figure 9 is a pattern of $\beta$-naphthaleneacetic acid for evaluating its physiological activity prepared in Example 3 by the method of this invention.

Figure 10 is a pattern of alloxan monohydrate for evaluating its physiological activity prepared in Example 4 by the method of this invention.

Figure 11 is a pattern of 6-aminonicotinamide for evaluating its physiological activity prepared in Example 4 by the method of this invention.

Figure 12 is a pattern of a mixture of $\beta$-indoleacetic acid and p-chlorophenoxy-isobutyric acid for evaluating its physiological activity prepared in Example 5 by the method of this invention.

### DESCRIPTION OF THE INVENTION

In the method disclosed in Japanese Patent Kokai No.130856 (1986) I formerly developed, in order to measure the fluctuations in liquid structure inherent to a substance being tested (limit cycle), enormous time and labor are required to determine the concentration that exactly corresponds to its limit cycle and to control the minute stream of heat to be applied to the test solution. Particularly when testing a physiologically inactive substance, which exhibits no limit cycle, one feels uneasy throughout the whole course of measurement.

Further studies have led me to find a method which represents the characteristics of a sample solution as a single value without the need to supply a minute stream of heat to the test solution nor to record the changes in characteristics with time.

I considered that minute heat sources of molecular scale exist in living bodies, that the heat radiations therefrom affect thermal vibrations and rotational movements of molecules of substances involved and hence have effects upon the fluctuations in their liquid structure, and that such fluctuations in liquid structure are directly related to the physiological activity of these substances. It was discovered that, since these fluctuations take shape as light scattering, one can evaluate the physiological activity of a substance by taking the degree of expansion of scattered light (that is, the size of transmitted laser beam) as the index of light scattering, plotting this beam size against concentration of the substance, and judging from the pattern of graph thus obtained. This invention was accomplished based on these findings. Thus this invention relates to a method for evaluating physiological activity of substances, which comprises placing test solutions (prepared by dissolving a substance to be tested in a buffer solution at different concentrations) and said buffer solution separately in a cell made of a material that is pervious to laser rays; transmitting a laser beam through each of the solutions placed in the cell while it is exposed to thermal radiation from an object held at a temperature higher than that of the test solution; measuring the intensity of transmitted laser beam while displacing a photoreceiver in the lateral direction toward the laser beam in a range within an angle of about 1.5° to the transmitted beam until a sharp rise in the intensity of scattered light is observed, whereby the size of transmitted laser beam expanded as a result of scattering is determined as the relative position of the photoreceiver at which the intensity of scattered light reaches a predetermined level; plotting the relative position of photoreceiver thus obtained against the concentration of substance being tested; and evaluating the physiological activity of said substance from the pattern of graph obtained above. This invention also relates to an apparatus for evaluating physiological activity of substances, which comprises a cell made of a material pervious to laser rays and having a space for test solution therein, and a radiation-heat supplying bath having a radiation-heat emitting wall surrounding the cell and provided with laser-beam passageways in the front and at the rear of the space for test solution, said cell being supported adiabatically against said radiation-heat emitting wall.

In the method of this invention, the substance being tested is dissolved in a buffer solution before use preferably at concentrations from $10^{-2}$ to $10^{-11}$ M. The buffer solution itself (not containing that substance) is also used in the test.

Each of the test solutions thus prepared or the buffer solution is placed in a cell about 1mm thick made of a material pervious to laser rays, such as pyrex glass.

The loaded cell is immersed in a warmer material (for example, in warm water) at its lower part and is surrounded by a metal wall painted black so as to receive radiation heat from said metal wall, wherein the temperature of said fluid is maintained one to a few degrees (in centigrade) lower than that of the cell during measurement.

A laser beam is then allowed to pass through the solution in the cell, wherein a 5-mW helium/neon gas laser of the plane polarization type is preferably used because it does not disturb the liquid structure and allows detection of slight changes.

Transmission of the laser beam through the cell gives rise to scattered light corresponding to the liquid structure of the particular test solution, and the intensity of this scattered light changes with the concentration of the substance being tested. A phototransistor used for detection converts the changes in intensity of scattered light into electric signals, which are input to the Y-axis of an X-Y recorder. The relative position of the phototransistor, on the other hand, is input to its X-axis.

The phototransistor is arranged so as to move on the same horizontal plane with the laser beam. The intensity of scattered light (output from the phototransistor) is input to the Y-axis of the X-Y recorder while slowly displacing the transistor toward the laser beam. Separately, a differential transformer is connected to the phototransistor, and the change in distance between the phototransistor and the laser beam is input to the X-axis as the change in output voltage of the transformer. It is advisable that the cell is properly tilted against the beam to enhance scattering capacity.

A scattering curve (scattering profile) can thus be prepared. This method is similar to the procedure for X-ray diffraction analysis except the use of differential transformer. The position of phototransistor at which the curve crosses the upper-limit horizontal line on the X-Y recording sheet may represent the bulge of beam, more specifically, a position in expanded beam that shows a definite intensity Y or the wall of expanded beam having the same brightness. This value can be exactly determined as a voltage reading on the differential transformer. It tends to shift outward when the intensity of scattered light is larger and the scattering curve moves upward, but, with solutions of physiologically active substances, this is positioned according to concentrations more inwardly compared with the buffer solution used. With solutions of physiologically inactive substances, on the other hand, this is positioned more inwardly than that of buffer solution in almost all cases. This indicates that solutions of inactive substances cause less light scattering than the buffer solution, that is, these are more transparent to laser rays.

Graphs for evaluating the physiological activity of substances may be prepared according to the procedure given below.

The relative positions of phototransistor for test solutions of different concentrations with respect to that for the buffer solution are plotted against the concentration, and the pattern of graph thus obtained is used for evaluating the type of physiological activity of the particular substance tested. The graph closely reflects the changes in liquid structure, the changes in interactions between the molecules of solvent and substance being tested, or the mechanical changes in the liquid lattice movements when the concentration of the substance, that is, intermolecular distance of the substance is varied over a wide range. Through these changes, are clearly represented the characteristics of its molecular structure interacting with the molecules of water — expression of the individual character of the particular molecule through its liquid structure, which can be evaluated as structural fluctuations by measurement of light scattering.

It is said that both animal and plant cells work in essentially the same way; therefore, the method of this invention is applicable to physiologically active substances contained in a wide range of living things. For example, plant hormones and herbicides are both plant growth regulating substances and are also common to teratogen in terms of cell differentiation, and hence, all of these substances can be evaluated in the same way by the method of this invention.

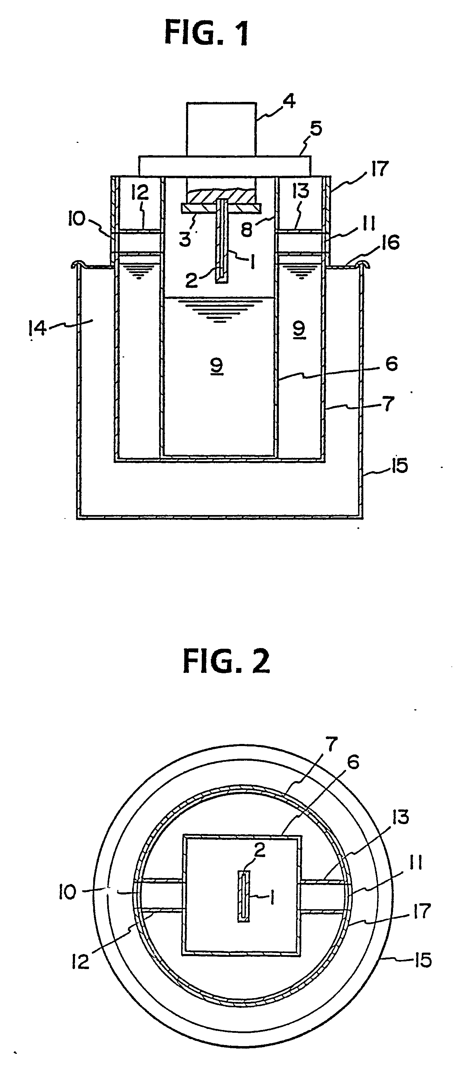

The apparatus of this invention is then detailed below while referring to Figures 1 and 2.

It consists of (1) a cell and cell holder assembly, in which a cell 1 containing the space for test solution 2 is fixed by the plastic plate holder 3, which in turn is fitted into the plastic cylindrical holder 4 provided with a member 5 which serves to retain said plastic cylinder and to adjust the direction of cell; and (2) a radiation-heat supplying assembly, in which a cylindrical cistern, with its bottom and lower half being wrapped with heat-insulating plastic foam 14, is divided into two separate sections (the inner, first water bath 6 and the outer, second water bath 7) by a radiation-heat emitting wall 8 (a square cylinder with its interior painted black) provided at the center of said cylindrical cistern, the first water bath 6 is provided with laser-beam passageway tubes 12 and 13 at positions just opposite to cell 1, the second water bath 7 is provided with laser-beam passageway ports 10 and 11 at positions just in front of 12 and 13, the upper wall of the second water bath is protected with a heat-insulating material 17, and heat-source water 9 is put in the first and second water baths; the member 5 in said cell and cell holder assembly being mounted on the top of metal wall 8 in said radiation-heat supplying assembly.

## Description of the Preferred Embodiments

The following Examples will further illustrate the method of this invention and the apparatus for the same.

### Example 1

(a) An aqueous solution of $\beta$-indoleacetic acid (a plant growth hormone) ($10^{-2}$ M, pH 4.72, 24°C) was put in the space for test solution 1mm wide 2 provided at the center of glass cell 1 40mm long, 24mm wide and 5mm thick, and the cell was also held at 24°C. The temperature of heat-source water was maintained at 26°C and accordingly the radiation-heat emitting metal wall 8 was held at about 26°C. The cell was inserted into the plastic plate holder 3 and mounted on the top of the first water bath 6 together with the plastic cylindrical holder 4. The cell was tilted by controlling the member 5 so that the reflected spot of laser beam will be at a position 5mm apart from the round slit, provided 30cm ahead of the cell, and on the side opposite to the phototransistor, thereby enhancing the intensity of scattered light.

A point 62cm behind the cell and about 5 cm apart from the beam is away from the beam by an angle of about 1.4° (an angle less than 1.5°) with respect to the beam passing point at the cell. Taking this point as origin, the phototransistor was displaced toward the laser beam to make a scattering profile on an X-Y recorder. The phototransitor was stopped when the recording pen reached the upper-limit horizontal line of the recording sheet, and this position of phototransistor was read as V value on a digital voltmeter. Similar experiments were repeated for aqueous solutions of $\beta$-indoleacetic acid (IAA) with concentrations of $10^{-3}$ M, $10^{-4}$ M, $10^{-5}$ M, $10^{-6}$ M, $10^{-7}$ M, $10^{-8}$ M,

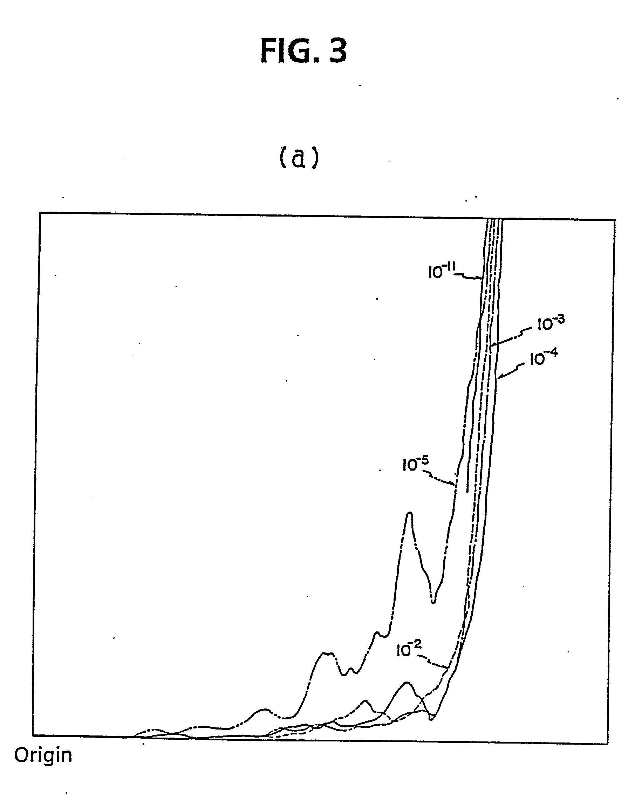

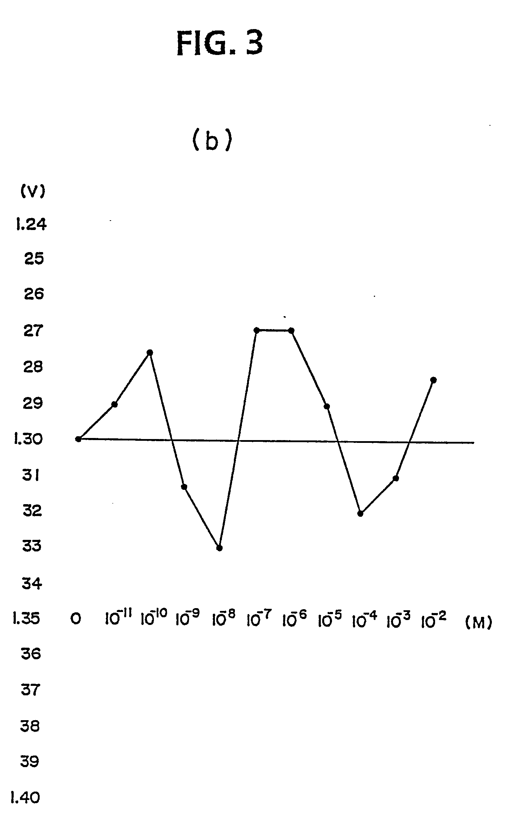

$10^{-9}$ M, $10^{-10}$ M and $10^{-11}$ M, and for the buffer solution (0 M). Scattering profiles for concentrations of $10^{-2}$ M, $10^{-3}$ M, $10^{-4}$ M, $10^{-5}$ M and $10^{-11}$ M are shown in Figure 3(a). Figure 3(b) is a graph in which the values V obtained above are plotted against concentration.

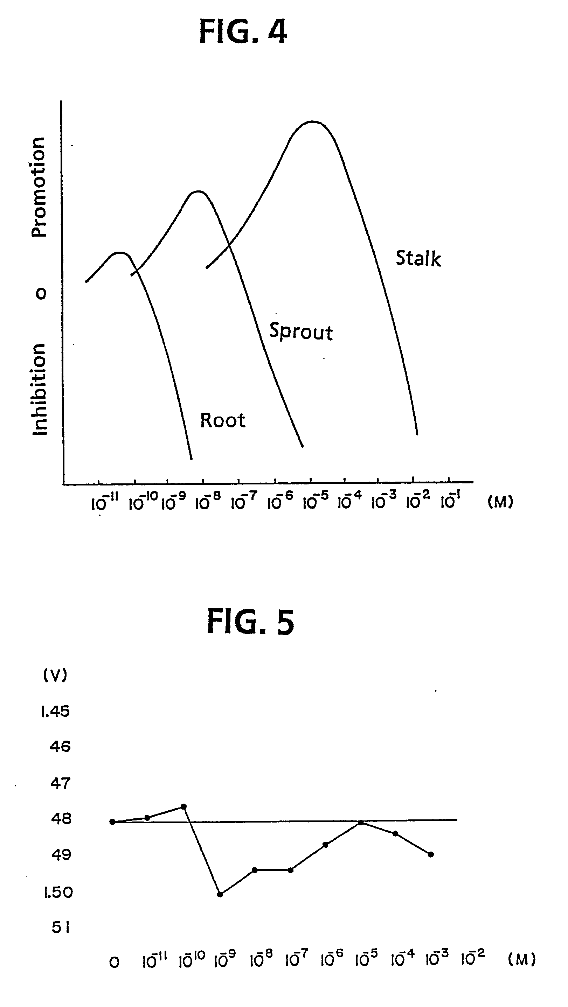

In this graph, a line running downward (increasing V value) indicates the phototransister coming close to the laser beam, that is, reducing size of the laser beam. In short, the upper areas of the figure represent larger beam size (namely, higher degree of light scattering). When the value for the buffer solution is taken as 0 level, the areas lower than this level show thinner laser beam (i.e., higher transparency of test solution) compared with the buffer solution. Two maximum peaks are observed for IAA as can be seen from Figure 3(b), but three peaks could exist if an experiment could be carried out at a concentration of $10^{-1}$ M. The positions of maximum peaks can be shifted by pH control, approaching Thimann's curves shown in Figure 4, the curves prepared by Thimann (1937) who applied IAA to plants at concentrations raging from $10^{-2}$ to $10^{-11}$ M and investigated its effect upon their growth (promotion and inhibition). The graph of Figure 3(b), in which the curve runs up and down across the 0 level, is very similar to Thimann's curves (Figure 4), and this pattern is common to physiologically active substances and may be taken as the measure for physiological activity.

(b) A similar test was conducted with indolecarboxylic acid which is a physiologically inactive substance (pH of buffer solution: 4.75, temperature of test solution in the cell: 19°C, temperature of metal wall: 23°C). The result is shown in Figure 5.

As may be seen from the figure, the curve lies for the most part below the 0 level, and this pattern was taken as the measure for physiologically inactive substances.

As is apparent from the results obtained above, difference in physiological activity due to a slight difference in molecular structure (a slight difference in the length of side chain) is clearly reflected in the pattern of graphs measured with a physical apparatus.

There was no significant difference in experimental conditions (pH, temperature of test solution and temperature of metal wall) between the two experiments (a) and (b).

Example 2

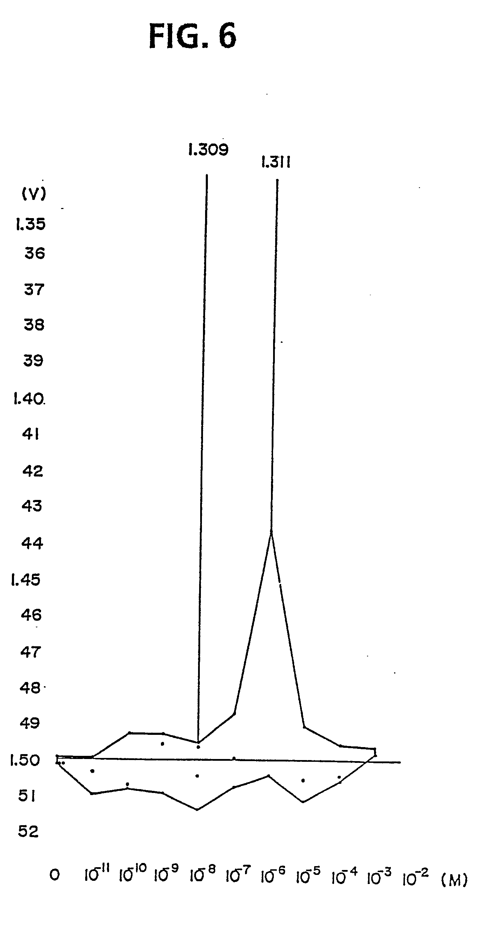

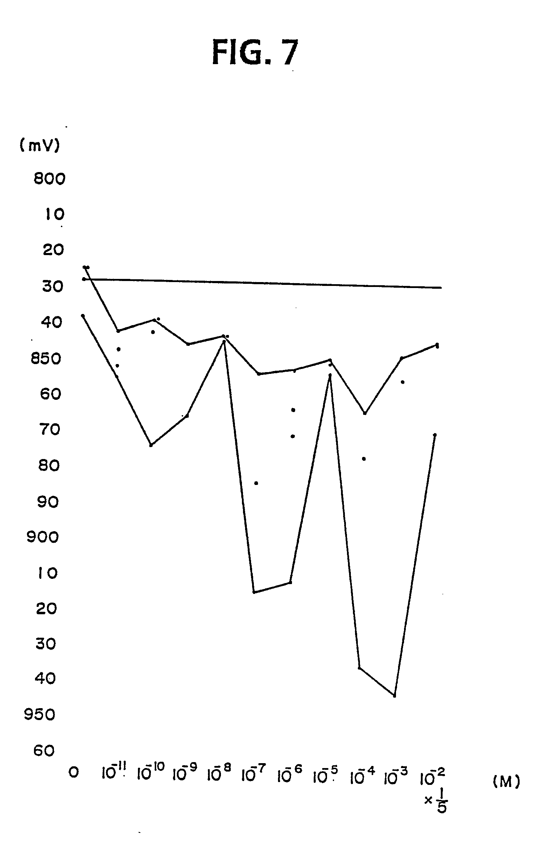

2,4,5-Trichlophenoxyacetic acid (2,4,5-T), which is a plant growth regulater and a herbicide, and 2,4,6-trichlorophenoxyacetic acid (2,4,6-T), which is a physiologically inactive substance, were tested in the same manner using the same apparatus as in Example 1(a). The results are shown in Figures 6 and 7, respectively. What is to be noticed here is that, in Figure 7, the values on the Y-axis are expressed in a different way because a differential transformer of different type was used, but this offers no problem for correct evaluation of physiological activity. In each of Figures 6 and 7, two curves were prepared by connecting together the highest V values at different concentrations, and by connecting the lowest V values as well, to show the distribution of the data obtained. These exhibit very characteristic patterns, but the standard for discriminating physiological activity and inactivity described in Example 1 also applies to this case. Although the difference in molecular structure between 2,4,5-T and 2,4,6-T is very small, the physiological activity and inactivity of these compounds can be clearly judged from the graphs.

In case of toxicity, the graph must have mode of activity and must also represent peculiar mode in degree of distribution of observed data, that is extreme variation in the degree of distribution against concentrations. Figure 6 is the example.

Example 3

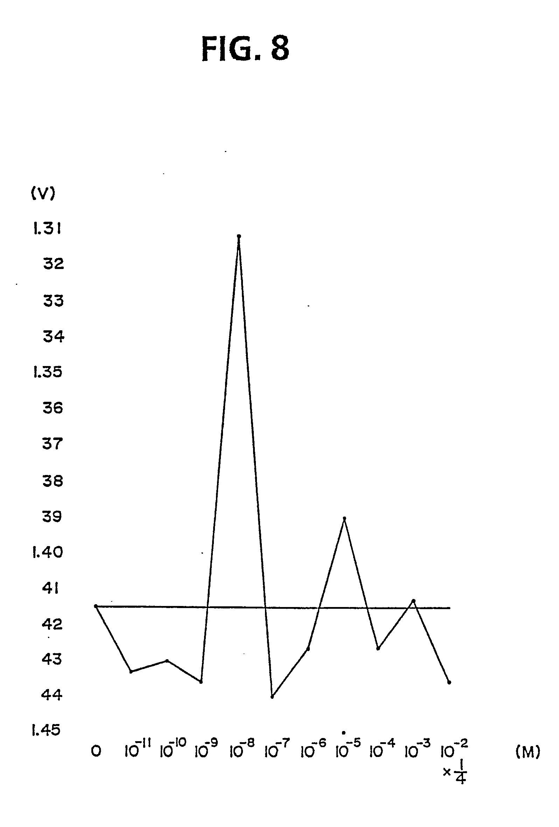

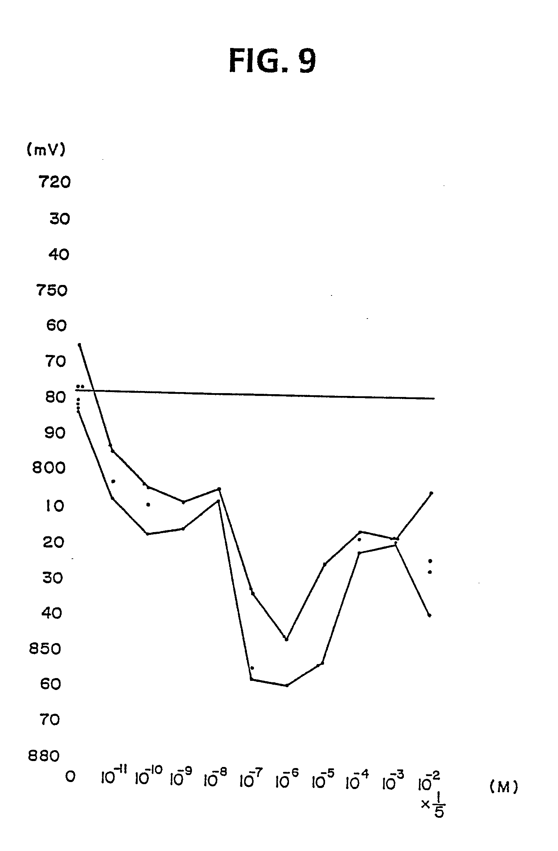

α-Naphthaleneacetic acid (α-NAA), which is a plant growth regulator, and β-naphthaleneacetic acid (β-NAA), which is a physiologically inactive substance, were tested in the same manner using the same apparatus as in Example 1(a). The results are shown in Figures 8 and 9, respectively. In the case of Figure 9, the values on the Y-axis are expressed in a different way because a differential transformer of different type was used, but this offers no problem for correct evaluation of physiological activity. Although the difference in molecular structure between the two compounds is very small, the difference in the pattern of graphs is conspicuous.

Example 4

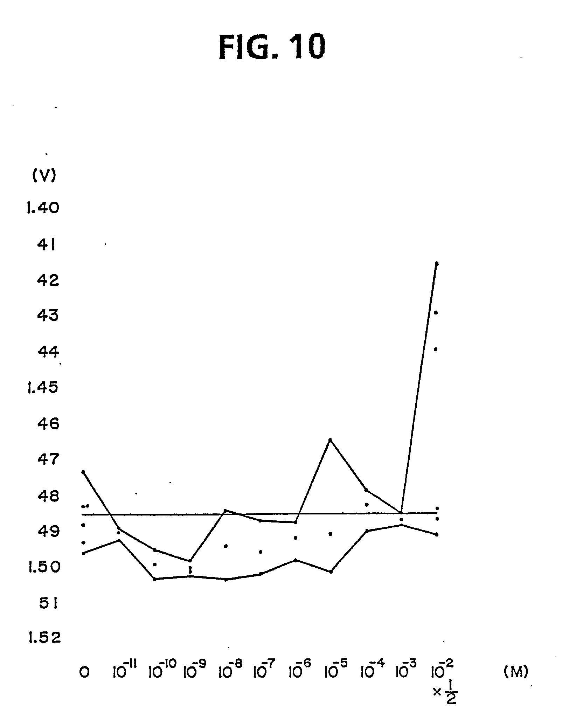

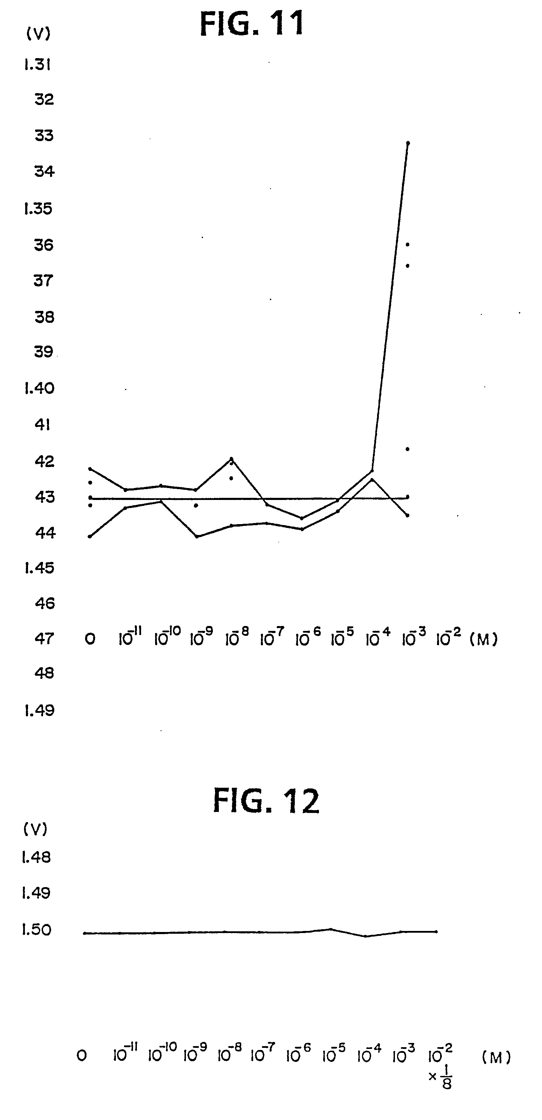

Alloxan monohydrate and 6-aminonicotinamide, both being teratogen, were tested in the same manner using the same apparatus as in Example 1-(a). The results are shown in Figures 10 and 11, respectively. The curves run up and down across the 0 level for the most part, clearly indicating that these substances are physiologically active.

In case of toxicity, the graph must have mode of activity and must also represent peculiar mode in degree of distribution of observed data, that is violent variation in the degree of distribution against concentrations. Figure 10 and Figure 11 are the examples.

Example 5

An aqueous solution of $\beta$-indoleacetic acid also containing the same concentration of p-chlorophenoxyisobutyric acid (an inibitor against $\beta$-indoleacetic acid) was tested in the same manner using the same apparatus as in Example 1(a). The result is shown in Figure 12. The curve runs nearly along the 0-level line, clearly indicating that the physiological activity of $\beta$-indoleacetic acid is almost completely inhibited. If conditions similar to those in living bodies are further created inside the cell, it would be possible to estimate optimum concentrations of a particular drug, synergistic effect in blended drugs, and preventive effect of a particular drug.

**Claims**

1. Method for evaluating physiological activity of substances, which comprises placing test solutions (prepared by dissolving a substance to be tested in a buffer solution at different concentrations) and said buffer solution separately in a cell made of a material that is pervious to laser rays; transmitting a laser beam through each of the solutions in the cell while it is exposed to thermal radiation from an object held at a temperature higher than that of the test solution; measuring the intensity of scattered light of laser beam while displacing a photoreceiver in the lateral direction toward the laser beam in a range within an angle of about $1.5°$ to the transmitted beam until a sharp rise in the intensity of scattered light is observed, whereby the size of transmitted laser beam expanded as a result of scattering is determined as the relative position of photoreceiver at which the intensity of scattered light reaches a predetermined level; plotting the relative position of photoreceiver thus obtained against the concentration of substance being tested; and evaluating the physiological activity of said substance from the pattern of graph prepared above.

2. An apparatus for evaluating physiological activity of substances, which comprises a cell made of a material pervious to laser rays and having a space for test solution therein, and a radiation-heat supplying bath having a radiation-heat emitting wall surrounding said cell and provided with laser-beam passageways in the front and at the rear of said test-solution space, said cell being supported adiabatically against said radiation-heat emitting wall.

# FIG. 1

# FIG. 2

# FIG. 3

## (a)

$10^{-11}$
$10^{-3}$
$10^{-4}$
$10^{-5}$
$10^{-2}$

Origin

# FIG. 3

## ( b )

(V)
1.24
25
26
27
28
29
1.30
31
32
33
34
1.35    O    $10^{-11}$  $10^{-10}$  $10^{-9}$  $10^{-8}$  $10^{-7}$  $10^{-6}$  $10^{-5}$  $10^{-4}$  $10^{-3}$  $10^{-2}$   (M)
36
37
38
39
1.40

# FIG. 4

# FIG. 5

# FIG. 6

# FIG. 7

# FIG. 8

# FIG. 9

(mV)

720

30

40

750

60

70

80

90

800

10

20

30

40

850

60

70

880

0    $10^{-11}$ $10^{-10}$ $10^{-9}$  $10^{-8}$  $10^{-7}$  $10^{-6}$  $10^{-5}$  $10^{-4}$  $10^{-3}$  $10^{-2}$     (M)

$\times \frac{1}{5}$

EP 0 322 477 A1

# FIG. 10

# FIG. 11

(V)

1.31

32

33

34

1.35

36

37

38

39

1.40

41

42

43

44

1.45

46

47   O    $10^{-11}$  $10^{-10}$  $10^{-9}$  $10^{-8}$  $10^{-7}$  $10^{-6}$  $10^{-5}$  $10^{-4}$  $10^{-3}$  $10^{-2}$  (M)

48

1.49

# FIG. 12

(V)

1.48

1.49

1.50

O    $10^{-11}$  $10^{-10}$  $10^{-9}$  $10^{-8}$  $10^{-7}$  $10^{-6}$  $10^{-5}$  $10^{-4}$  $10^{-3}$  $10^{-2}$   (M)

$\times \frac{1}{8}$

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | PATENT ABSTRACTS OF JAPAN, vol. 10, no. 324 (P-512)[2380], 5th November 1986; & JP - A - 61 130 856 (HIROAKI INOUE) 18-06-1986 (Cat. D) | 1 | G 01 N 21/51 |
| A | US-A-4 061 543 (C.P. BEAN et al.) * column 1, lines 4-30; column 3, lines 42-56 * | 1 | |
| A | DE-A-2 342 171 (PFIZER INC.) * pages 1-5 * | 1 | |
| Y | EP-A-0 064 110 (A.M.T.E.C.) * page 3, line 5 - page 4, line 3; page 6, lines 24-30 * | 1 | |
| A | US-A-4 355 897 (W.I. KAYE) * column 1, lines 63-67 * | 1 | |
| Y | US-A-3 730 842 (P.J. WYATT et al.) * column 1, line 49 - column 3, line 36 * | 1 | TECHNICAL FIELDS SEARCHED (Int. Cl.4) |
| A | POLYMER LETTERS, vol. 9, 1971, pages 695-699; W. KAYE et al.: "Light scattering measurements on liquids at small angles" | | G 01 N 21/00 G 01 N 33/00 C 12 Q 1/00 |

The present search report has been drawn up for all claims

| Place of search | Date of completion of the search | Examiner |
|---|---|---|
| BERLIN | 17-08-1988 | BRISON O.P. |

EPO FORM 1503 03.82 (P0401)